# EUROPEAN PATENT SPECIFICATION

(11) **EP 2 170 170 B1**
(45) Date of publication and mention of the grant of the patent: **02.12.2015**
(21) Application number: 08762439.1
(22) Date of filing: 20.06.2008
(51) Int. Cl.: A61B 5/15, A61B 5/00

(54) **LANCING DEVICES**
LANZETTENVORRICHTUNGEN
AUTOPIQUEUR

(30) Priority: 22.06.2007 GB 0712107
(43) Date of publication of application: 07.04.2010
(73) Proprietor: OWEN MUMFORD LIMITED, Woodstock, Oxford OX20 ITU (GB)
(72) Inventor: EVANS, Timothy, Simon, Oxfordshire OX18 2JY (GB)
(74) Representative: Wynne-Jones, Laine and James LLP
(86) International application number: PCT/GB2008/002126
(87) International publication number: WO 2009/001049

(56) References cited:
- EP-A- 1 166 719
- JP-A- 2000 166 902
- US-A1- 2003 109 895
- US-A1- 2004 254 599

## Description

This invention relates to lancing devices and in particular, but not exclusively, to such devices designed to receive a disposable or replaceable lancet for skin pricking.

In applications where a user needs to prick the skin regularly to draw a bead of blood for test purposes (e.g. blood glucose level), it is common to provide a lancing device comprising a housing containing a lancet drive mechanism and a lancet holder which receives in use a disposable lancet. The user loads a disposable lancet into the device, cocks it and then triggers the device to cause the lancet to prick the skin. After use, the lancet is disposed of to prevent contamination or infection. It is also known to provide the lancet with a protective cap which is moulded over the lancet needle, so that the lancet can be handled safely prior to loading it in the lancing device and to keep it sterile. Once loaded, the cap can be twisted off to expose the needle. After the device has been fired, the cap can be reattached to the lancet to cover the needle prior to disposal.

It is therefore important to provide some means of preventing the lancet rotating in the holder when the cap is twisted off. Also, for quality control and product liability purposes a lancing device should only used with disposable lancets specifically designed for use therewith. With the small dimensions of the lancet and the lancet holder it can be difficult for non-technical patients or those with limited vision or impaired dexterity to correctly align with the lancet holder a lancet having a non-circular shaft. Likewise, it is important to ensure that the lancet does not jam in the lancet holder as this could encourage the user to use excessive force, possibly exposing the lancet needle prematurely or bending the tip whilst still capped, or in the lancet being too far forward in the lancet holder thereby leading to malfunction.

US2004/25499 discloses in Examples 3 and 4 lancing arrangements in which the lancet and the lancet holder are keyed so that they fit together in a specific orientation during assembly. If the lancet and lancet carrier are not properly oriented they will not fit together.

US2003/0109895 discloses another arrangement in which the lancet and the lancet holder are complementarily keyed.

EP1166719 discloses an arrangement in which a lancet is received in a lancet holder by a coupling mechanism that comprises matching coupling elements.

JP2000166902 discloses an arrangement comprising a lancing device in which a lancet is loaded into a lancet holder.

Accordingly, in one aspect, this invention provides a lancing device comprising a housing containing a lancet drive mechanism and a lancet holder for receiving in use a disposable lancet introduced rearwardly, said lancet having an elongate non-circular shaft, said lancet holder having a wall region defining an elongate space for receiving the shaft of said lancet, the lancet holder having at least one internally projecting rib for resisting rotation of a lancet when fully home in said lancet holder, and the internally projecting rib having at its forward end a pointed forward deflecting region which is pointed in shape to urge said lancet shaft angularly about its longitudinal axis to orient said lancet such that said lancet in use is aligned angularly with said elongate space during insertion.

The terms 'forward', 'forwardly' etc in connection with the lancing device connection refer to the direction of travel of the lancet when moving to prick.

The lancet holder preferably comprises three internally projecting ribs extending longitudinally to cooperate with the elongate flute regions on the lancet. In another arrangement said lancet may have a shaft of rectangular cross-section, for example of the type shown in US Re35803 or US5318584.

Preferably said radial flanges are equiangularly spaced.

In an embodiment the lancing device is configured to receive a lancet having a non-circular shaft comprising two or more equiangularly spaced radial flanges extending longitudinally to define a plurality of intervening relieved flute regions, and the lancet holder includes correspondingly spaced projecting ribs. The ribs may taper in width and radial dimension in a forward region thereof to define said pointed deflecting region.

The lancet holder preferably includes resilient detent means for releasably retaining a lancet in use, for example two diametrically opposed resilient detent means. The resilient detent means conveniently comprise a flexible tongue portion formed in the wall region of the holder.

Preferably the holder has an odd number of ribs, and one of said resilient detent means is aligned with, or forms part of, one of said ribs, and the diametrically opposed resilient detent is disposed in a gap between two of said ribs.

Whilst the invention has been described above, it extends to any inventive combination or sub-combination of the features set out above or in the following description.

The invention may be performed in various ways, and an embodiment thereof will now be described by way of example only, reference being made to the accompanying drawings in which:
Figure 1 is a cross sectional view of a prior art lancing device disclosed in published International patent application No. WO01/13794;
Figure 2 is a perspective view on a lancet holder of a lancing device of an embodiment of this invention, with a lancet loaded;
Figure 3 is a perspective view similar to Figure 2 but showing the lancet prior to insertion into the lancet holder;
Figure 4 is a detailed cross sectional view on the lancet holder of Figures 2 and 3, and
Figures 5 and 6 are perspective views of the lancet and lancet holder of Figures 2 to 4, from different aspects.

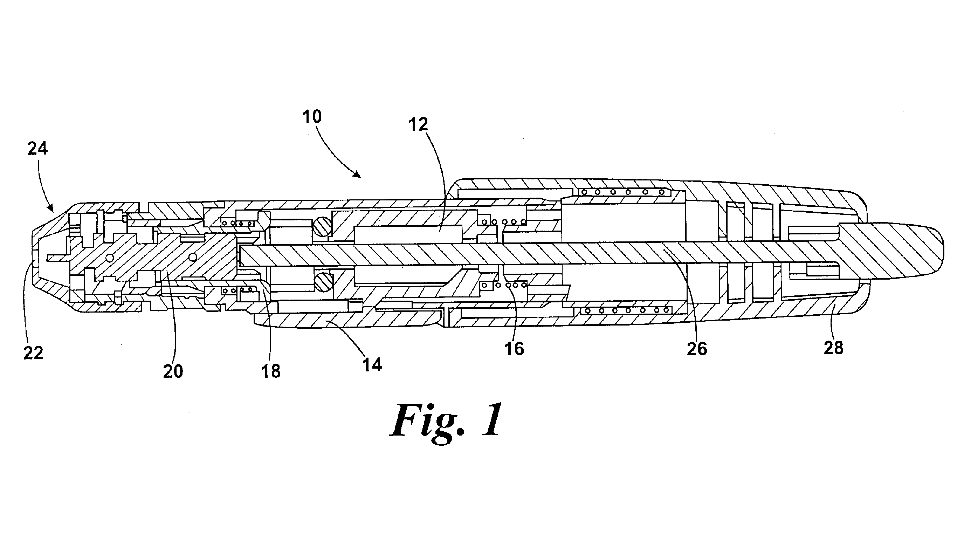

Referring initially to Figure 1 there is shown a lancing device of the type described in WO01/13794. This device comprises a barrel 10 in which a hammer 12 released by a trigger mechanism 14 can be shot forwards by a compressed spring 16 to impact on the rear end of a lancet holder 18 to drive it forward so a lancet 20 held therein can momentarily project its needle tip through an aperture 22 in a nose piece 24. After firing, the nose piece and the attached collar may be removed and the lancet 20 pushed forwardly out of the lancet holder 18 by an ejector rod 26. A new lancet 20 can be inserted following removal of the nose piece 24 and pushed into the lancet holder 18. In this particular arrangement, the hammer is re-cocked by pulling back a sleeve 28 on the housing which pulls the hammer 12 back to its cocked position as set out in more detail in WO01/13794.

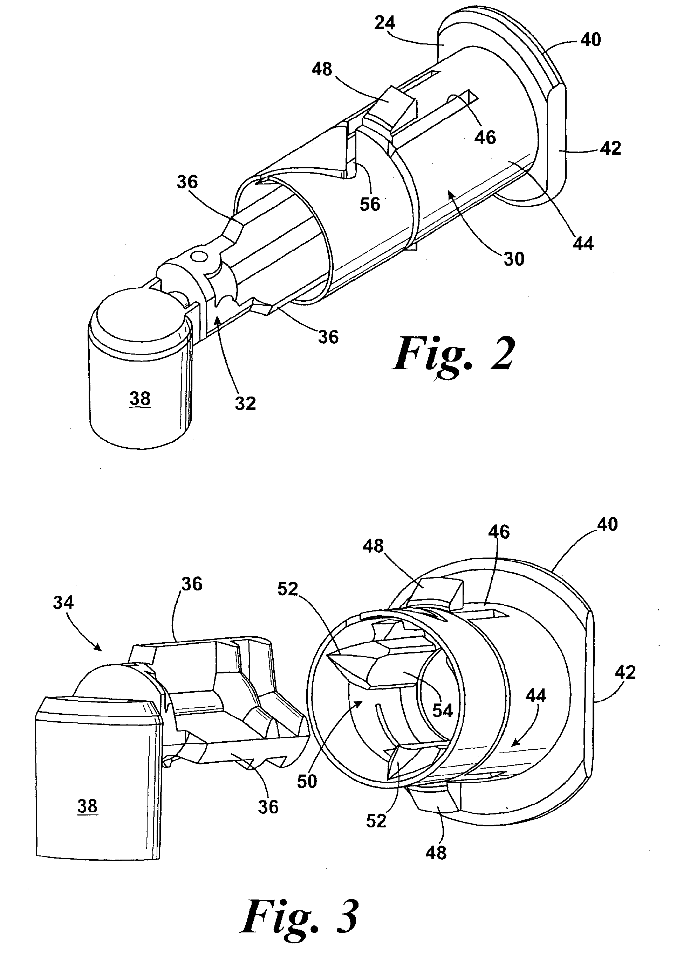

In the illustrated embodiment of this invention, the lancet holder 18 and lancet 20 are replaced by the lancet holder 30 and lancet 32 as shown in Figures 2 to 6. Otherwise the lancing device operates substantially as previously described, although of course the invention may be used in any of a wide range of different lancing devices where a lancet is inserted into a holder.

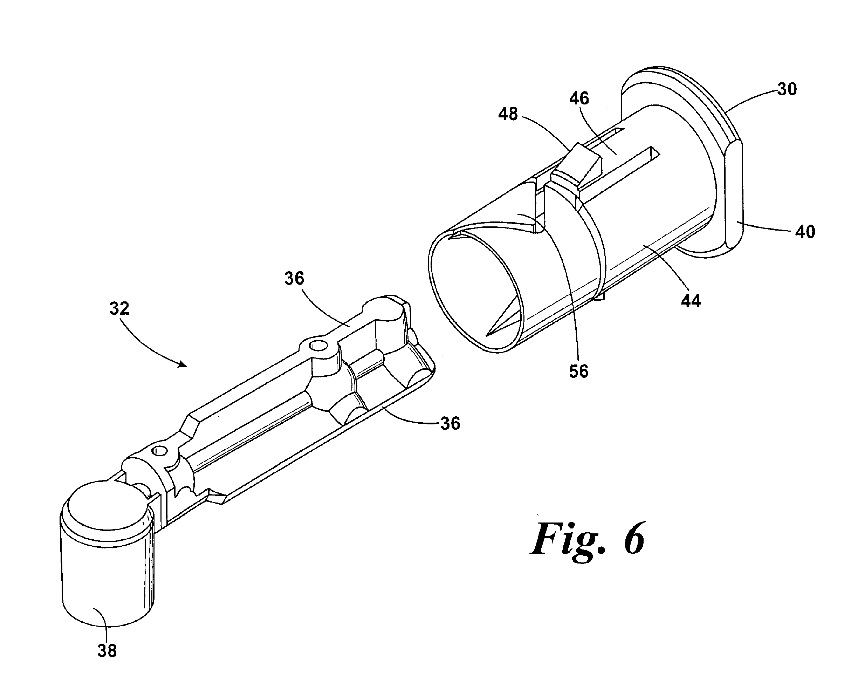

Referring now to Figures 2 to 6, the lancet comprises an elongate shaft 34 comprising three radial flanges 36 equiangularly spaced to define flute regions between them. The lancet has a cap 38 integrally moulded over the tip, the cap being removable by twisting. The lancet holder has a rear outwardly turned rim 40 for receiving the impact of the hammer, the rim 40 having flats 42 to prevent it rotating within the housing. Forwardly of the rim 40 there is a generally cylindrical sleeve 44 having two diametrically opposed tongues 46 capable of radial resilient movement and having on their forward ends barbs 48. The barbs 48 cooperate with a forwardly facing shoulder on the front of the housing 10 of the lancing device to limit rearward movement of the lancet body. Internally of the cylindrical sleeve 44 there are provided three equi-spaced guide ribs/deflectors 50. Each guide rib/deflector 50 comprises a pointed alignment region 52 forwardly of a key rib 54. A V 56 is cut in the forward end of the sleeve and communicates with one of the slots defining the upper tongue as seen in Figure 6.

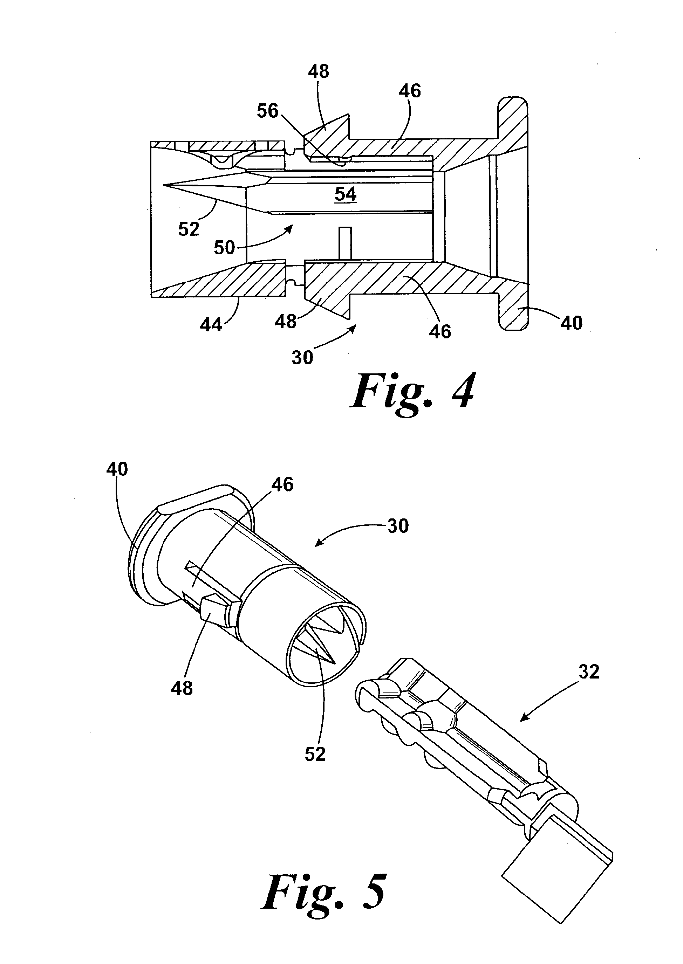

The guide ribs/deflectors 50 are disposed round the sleeve such that one of them is angularly aligned with one of the resilient teeth 46, with the other resilient tooth 46 lying midway between two guide ribs/deflectors 50. The tooth 46 lying between the two guide ribs/deflectors is provided with an inwardly directed pip 56, (see Figure 4).

In use, when a lancet 32 is offered up to the lancet holder, in any configuration, if the ribs 36 are not aligned with the gaps between the guide ribs/deflectors 50 on the holder 50, the pointed alignment regions 52 will nudge the lancet angularly to effect such alignment. As the lancet is pushed home into the lancet holder, the pip 56 on the upper tooth (as seen in Figure 4) rides on one of the ribs 36 to provide a frictional hold.

When the lancet is fully home, the cap may be twisted off and rotation of the lancet relative to the holder is prevented by inter-engagement of the radial flanges 36 with the guide ribs/deflectors 50 and by the flats 42 on the lancet holder engaging corresponding surfaces on the interior of the housing of the lancing device.

## Claims

1. A lancing device comprising a housing (10) containing a lancet drive mechanism and a lancet holder (30) for receiving in use a disposable lancet (32), said lancet having an elongate non-circular shaft, said lancet holder (30) having a wall region (44) defining an elongate space for receiving the shaft of said lancet introduced rearwardly, the lancet holder having at least one internally projecting rib (50) for resisting rotation of a lancet when fully home in said lancet holder (30), and **characterised in that** the internally projecting rib has at its forward end a forward deflecting region which is pointed in shape (52) to urge said lancet shaft angularly about its longitudinal axis to orient said lancet such that said lancet (32) in use is aligned angularly with said elongate space during insertion.

2. A lancing device according to Claim 1, wherein said lancet holder (30) comprises three internally projecting ribs (50) extending longitudinally to cooperate in use with respective elongate relieved flute regions on the disposable lancet.

3. A lancet device according to Claim 2, wherein said internally projecting ribs (50) are equispaced.

4. A lancing device according to Claim 2 or Claim 3, wherein said internally projecting ribs (50) taper in width and radial dimension in a forward region thereof to define said pointed deflecting region (52).

5. A lancing device according to any preceding claim, wherein said lancet holder (30) includes resilient detent means (46, 56) for releasably retaining a lancet in use.

6. A lancing device according to Claim 5 wherein said lancet holder (30) comprises two diametrically opposed resilient detent means (46).

7. A lancing device according to Claim 5 or Claim 6, wherein said resilient detent means (46) comprise a flexible tongue portion formed in the wall region of the holder.

8. A lancing device according to Claim 6 or Claim 7, wherein the holder has an odd number of ribs (50) and wherein one of said resilient detent means (46) is aligned with, or forms part of, one of said ribs, and the diametrically opposed resilient detent (46) is disposed in a gap between two of said ribs (50).

9. A lancing device according to any preceding claim, configured to receive a lancet (20) having a non-circular shaft (34) comprising two or more equiangularly spaced radial flanges (36) extending longitudinally to define a plurality of intervening relieved flute regions, with the lancet holder including correspondingly spaced projecting ribs (50).

## Patentansprüche

1. Lanzettenvorrichtung mit einem Gehäuse (10), aufweisend einen Lazettensteuermechanismus und einen Lanzettenhalter (30) zum Aufnehmen einer Einweglanzette (32) beim Gebrauch, wobei die Lanzette einen länglichen nicht-kreisförmigen Schaft aufweist, wobei der Lanzettenhalter (30) einen Wandabschnitt (30) aufweist, der einen länglichen Raum zum Aufnehmen des Schaftes der rückwärts eingeführten Lanzette begrenzt, wobei der Lanzettenhalter zumindest eine einwärts vorstehende Rippe (50) zum Gegenhalten gegen eine Rotation der Lanzette aufweist, wenn sie vollständig in dem Lanzettenhalter (30) eingeführt ist, und **gekennzeichnet dadurch, dass** die einwärts vorstehende Rippe an ihrem vorderen Ende einen vorderen Ablenkabschnitt aufweist, die spitzförmig (52) ausgebildet ist, um den Lanzettenschaft in Bezug auf seine Längsachse schräg zu veranlassen, die Lanzette derart zu orientieren, dass die Lanzette (32) beim Gebrauch schräg zu dem länglichen Raum während des Einfügen ausgerichtet ist.

2. Lanzettenvorrichtung nach Anspruch 1, wobei der Lanzettenhalter (30) drei einwärts vorstehende Rippen (50) aufweist, die sich der Länge nach erstecken, um beim Gebrauch mit jeweiligen länglichen, sich verjüngenden Nutenabschnitten auf den Eingweglanzetten zusammenwirken.

3. Lanzettenvorrichtung nach Anspruch 2, wobei die einwärts vorstehende Rippen (50) äquidistant angeordnet sind.

4. Lanzettenvorrichtung nach Anspruch 2 oder Anspruch 3, wobei die einwärts vorstehende Rippen (50) sich in Breite und radialer Abmessung in einem vorderen Abschnitt hiervon verjüngen, um den spitzen Ablenkabschnitt (52) zu definieren.

5. Lanzettenvorrichtung nach einem der vorstehenden Ansprüche, wobei der Lanzettenhalter (30) elastische Rastmittel (46, 56) zum lösbaren Festhalten einer Lanzette beim Gebrauch aufweist.

6. Lanzettenvorrichtung nach Anspruch 5, wobei der Lanzettenhalter (30) zwei diametral sich gegenüberliegende elastische Rastmittel (46) aufweist.

7. Lanzettenvorrichtung nach Anspruch 5 oder Anspruch 6, wobei die elastischen Rastmittel (48) einen flexiblen Zungenabschnitt aufweisen, der in dem Wandabschnitt des Halters gebildet ist.

8. Lanzettenvorrichtung nach Anspruch 6 oder Anspruch 7, wobei der Halter eine ungerade Anzahl von Rippen (50) aufweist, und wobei eine der elastischen Rastmittel (46) ausgerichtet ist an oder Teil einer der Rippen ist, und die diametral sich gegenüberliegende elastische Rastmittel (46) in einem Spalt zwischen zwei Rippen (50) angeordnet sind.

9. Lanzettenvorrichtung nach einem der vorstehenden Ansprüche, ausgebildet zum Aufnehmen einer Lanzette (20) mit einem nicht-kreisförmigen Schaft (34), der zwei oder mehrere gleichwinkelig beabstandete radiale Flansche (36) aufweist, die sich der Länge nach erstrecken, um eine Mehrzahl dazwischenliegender sich verjüngender Nutenabschnitten zu definieren, und mit dem Lanzettenhalter, der korrespondierend beabstandet angeordnete vorstehende Rippen (50) aufweist.

## Revendications

1. Dispositif autopiqueur comprenant un boîtier (10) contenant un mécanisme d'entraînement de lancette et un support de lancette (30) pour recevoir, en utilisation, une lancette jetable (32), ladite lancette ayant une tige non circulaire allongée, ledit support de lancette (30) ayant une région de paroi (44) définissant un espace allongé pour recevoir la tige de ladite lancette introduite par l'arrière, le support de lancette ayant au moins une nervure en saillie vers l'intérieur (50) pour résister à la rotation d'une lancette lorsqu'elle est entièrement reçue dans ledit support de lancette (30), et **caractérisé par le fait que** la nervure en saillie vers l'intérieur a, à son extrémité avant, une région de déviation avant qui a une forme pointue (52) pour pousser ladite tige de lancette angulairement autour de son axe longitudinal pour orienter ladite lancette de telle sorte que ladite lancette (32), en utilisation, est alignée angulairement avec ledit espace allongé pendant l'introduction.

2. Dispositif autopiqueur selon la revendication 1, dans lequel ledit support de lancette (30) comprend trois nervures en saillie vers l'intérieur (50) s'étendant longitudinalement pour coopérer, en utilisation, avec des régions de cannelure réduites allongées respectives sur la lancette jetable.

3. Dispositif autopiqueur selon la revendication 2, dans lequel lesdites nervures en saillie vers l'intérieur (50) sont espacées de manière équidistante.

4. Dispositif autopiqueur selon l'une des revendications 2 ou 3, dans lequel lesdites nervures en vers l'intérieur (50) s'effilent en largeur et en dimension radiale dans une région avant de celles-ci pour définir ladite région de déviation pointue (52).

5. Dispositif autopiqueur selon l'une quelconque des revendications précédentes, dans lequel ledit support de lancette (30) comprend des moyens d'arrêt élastique (46, 56) pour retenir de façon libérable une lancette en utilisation.

6. Dispositif autopiqueur selon la revendication 5, dans lequel ledit support de lancette (30) comprend deux moyens d'arrêt élastiques diamétralement opposés (46).

7. Dispositif autopiqueur selon l'une des revendications 5 ou 6, dans lequel lesdits moyens d'arrêt élastiques (46) comprennent une partie languette souple formée dans la région de paroi du support.

8. Dispositif autopiqueur selon l'une des revendications 6 ou 7, dans lequel le support a un nombre impair de nervures (50) et dans lequel l'un desdits moyens d'arrêt élastiques (46) est aligné avec ou fait partie de l'une desdites nervures, et le moyen d'arrêt élastique diamétralement opposé (46) est disposé dans un intervalle entre deux desdites nervures (50).

9. Dispositif autopiqueur selon l'une quelconque des revendications précédentes, configuré pour recevoir une lancette (20) ayant une tige non circulaire (34) comprenant au moins deux brides radiales espacées équiangulairement (36) s'étendant longitudinalement pour définir une pluralité de régions de cannelure réduites intercalées, avec le support de lancette comprenant des nervures en saillie espacées de façon correspondante (50).
